# EUROPÄISCHE PATENTANMELDUNG

(11) **EP 1 413 528 A2**
(43) Veröffentlichungstag der Anmeldung: **28.04.2004**
(21) Anmeldenummer: 03027354.4
(22) Anmeldetag: 24.05.2000
(51) Int. Cl.: B65D 83/14, B21D 51/24, A61M 15/00, C22C 38/44, C22C 38/42, C22C 30/00

(54) **Edelstahlkanister fur treibgasbetriebene Dosieraerosole**

(30) Priorität: 26.05.1999 DE 19924098
(62) Teilanmeldung aus: 00943730.2
(71) Anmelder: BOEHRINGER INGELHEIM PHARMA GMBH & CO. KG, 55216 Ingelheim am Rhein (DE)
(72) Erfinder: Nagel, Juergen, 55543 Kreuznach (DE); Hoelz, Hubert, 55413 Oberheimbach (DE); Vega, Julio César, 1429 Capital Federal Buenos Aires (AR); Lostritto, Richard Thomas, Gaithersburg 20879-1844 Maryland (US)

(57) **Zusammenfassung**

Die vorliegende Erfindung betrifft korrionsbeständige Edelstahlkanister für treibmittelhaltige Aerosolformulierungen zur Verwendung in treibgasbetriebenen Inhalatoren.

## Beschreibung

Die vorliegende Erfindung betrifft korrosionsbeständige Edelstahlkanister für treibmittelhaltige Aerosolformulierungen zur Verwendung in treibgasbetriebenen Inhalatoren.

### Hintergrund der Erfindung

In treibmittelbetriebenen Inhalatoren werden die Wirkstoffe zusammen mit dem Treibmittel in patronenähnlichen Kanistern gelagert. Diese Kanister bestehen in der Regel aus einem Aluminiumbehälter, der mit einer Ventiltasse aus Aluminium verschlossen ist, in die ein Ventil eingebettet ist. Ein solcher Kanister kann dann wie eine Patrone in den Inhalator eingeführt und verbleibt dort entweder permanent oder wird nach Gebrauch gegen eine neue Patrone ausgetauscht. Seitdem Anfang der 90er Jahre auf der Konferenz von Rio de Janeiro Fluorchlorkohlenwasserstoffe (FCKW, CFC) wegen ihrer Ozon-zerstörenden Eigenschaften weltweit geächtet wurden, wird als Alternative die Verwendung von Fluorkohlenwasserstoffen (FKW, HFA) für die Verwendung in treibmittelbetriebenen Inhalatoren angestrebt. Die zur Zeit vielversprechendsten Vertreter sind TG 134a (1,1,2,2-Tetrafluorethan) und TG 227 (1,1,1,2,3,3,3-Heptafluorpropan). Dementsprechend müssen die bestehenden Applikationssysteme für die inhalative Therapie auf FCKW-freie Treibmittel umgestellt werden und neue Applikationssysteme und Wirkstoff- Formulierung entwickelt werden.

Überraschenderweise hat sich herausgestellt, daß Aluminiumkanister gegenüber Arzneistoff-Formulierungen mit Fluorkohlenwasserstoffen als Treibmittel nicht immer beständig sind, sondern in Abhängigkeit der Zusammensetzung der Formulierungen ein hohes Korrosionsrisiko aufweisen. Dies gilt insbesondere für Formulierungen, in denen Elektrolyte und/oder freie Ionen, besonders freie Halogenide vorliegen. In diesen Fällen wird das Aluminium angegriffen, so daß Aluminium als Mantelmaterial für die Kanister nicht verwendet werden kann. Ähnliche Unbeständigkeiten der Aluminiumkanister bei der Verwendung von Fluorkohlenwasserstoffen als Treibmittel werden beobachtet, wenn die Formulierungen saure oder basische Bestandteile enthalten; z.B. in Form der Wirkstoffe, der Zusatzstoffe, als Stabilisatoren, Surfactants, Geschmacksverstärker, Antioxidantien u.ä.

### Beschreibung der Erfindung

Es ist eine der Aufgaben der vorliegenden Erfindung, einen Kanister für treibmittelbetriebene Inhalatoren zu schaffen, der korrosionsbeständig ist gegenüber Wirkstoff-Formulierungen für die Inhalationstherapie mit einem Fluorkohlenwasserstoff als Treibmittel, der für die Verarbeitung und für den Gebrauch eine ausreichende Druck- und Bruchfestigkeit besitzt, der die Qualität der aufzubewahrenden Formulierungen gewährleistet und die anderen aus dem Stand der Technik bekannten Nachteile überwindet.

Eine weitere Aufgabe der Erfindung besteht darin, einen Kanister für treibmittelbetriebene Inhalatoren zu schaffen, dessen Behälter aus einem einzigen in sich homogenen Material besteht.

Überraschenderweise wurde gefunden, daß Kanister bestehend aus einem Behälter und einer Ventiltasse mit Ventil, bei denen zumindest der Behälter aus bestimmten Edelstahllegierungen besteht, die erfindungsgemäße Aufgabe lösen. Diese Legierungen beinhalten als Bestandteile Chrom (Cr), Nickel (Ni), Molybdän (Mo), Eisen (Fe) und Kohlenstoff (C). Solche Legierungen können zusätzlich Kupfer (Cu), Mangan (Mn) und Silicium (Si) enthalten. Bevorzugt besteht der Behälter aus einer der unten beschriebenen Legierungen.

Die Erfindung betrifft ferner die Verwendung eines derartigen Behälters bzw. Kanisters, bestehend aus einem Behälter und einer Ventiltasse mit Ventil in treibmittelbetriebenen Dosieraerosolen (Inhalatoren) und ein Verfahren zur Herstellung derselben.

Im folgenden wird die Erfindung anhand der Figuren 1 und 2 näher besprochen.

Fig. 1 zeigt den Kanister bestehend aus Behälter (2), Ventiltasse (8) und das Ventil (9) im Querschnitt.

Fig. 2 zeigt eine weitere Ausführungsform der Ventiltasse (8) und des Ventils (9) im Querschnitt.

Figur 1 zeigt den erfindungsgemäßen Kanister (1) im Querschnitt. Der Kanister (1) besteht aus einem Behälter (2) zur Aufnahme der Arzneistoff-Formulierung und einer Ventiltasse (8) mit Ventil (9). Die Form und Dimension des Kanisters entspricht den aus dem Stand der Technik bekannten Aluminiumkanistern.

Der erfindungsgemäße Behälter (2) besteht aus einer Legierung mit einem Anteil an
Eisen von 40,0 - 53,0%,
Nickel 23,0 - 28,0%,
Chrom 19,0 - 23,0%,
Molybdän 4,0 - 5,0%,
Mangan 0,0 - 2,0%,
Kupfer 1,0 - 2,0%,
Silicium 0,0 - 1,0%,
Phosphor 0,0 - 0,045%,
Schwefel 0,0 - 0,035% und
Kohlenstoff 0,0 - 0,020%.
Bei dieser Legierung handelt es sich um eine Legierung gemäß der Werkstoffnummer 1.4539 der Stahl-Eisen-Liste des Vereins deutscher Eisenhüttenleute.

Eine bevorzugte Legierung dieser Art weist folgende Zusammensetzung auf:
Chrom 19,0 - 21,0%,
Nickel 24,0 - 26,0%,
Molybdän 4,0 - 5,0%,
Kupfer 1,0 - 2,0%,
Mangan bis zu 2,0%,
Silicium bis zu 0,5% und
Kohlenstoff bis zu 0,02%, wobei der restliche Bestandteil im wesentlichen Eisen ist.

In einer nahezu identischen Alternativlegierung ist der Molybdängehalt auf 4,5-5,0% eingeschränkt.

In einer alternativen Ausführungsform besteht der erfindungsgemäße Behälter (2) aus einer Legierung gemäß der Werkstoffnummer 1.4404 der Stahl-Eisen-Liste des Vereins deutscher Eisenhüttenleute.

Die Zusammensetzung der Legierung ist:
Eisen von 60,0 - 72,0%,
Nickel 9,0 - 13,0%,
Chrom 17,0 - 21,0%,
Molybdän 2,0 - 3,0%,
Mangan 0,0 - 1,5%,
Silicium 0,0 - 1,5%,
Phosphor 0,0 - 0,04%,
Schwefel 0,0 - 0,04% und
Kohlenstoff 0 - 0,03%.

Eine weitere Ausführungsform des Behälters besteht aus einer Legierung der folgenden Zusammensetzung:
Chrom 16,5 - 18,5 %,
Nickel 11,0 - 14,0%,
Molybdän 2,0 - 2,5%,
Kohlenstoff maximal 0,03% und Eisen als restlichem Bestandteil.

Die genannten Legierungen sind derart, daß sie gegenüber verschiedenen verflüssigten Fluorkohlenwasserstoffen wie TG 134a (1,1,1,3-Tetrafluorkohlenwasserstoff) und TG 227 (1,1,1,2,3,3,3-Heptafluorpropan) korrosionsbeständig sind. Dazu zählen Treibgasformulierungen mit für die inhalative Therapie geeigneten Wirkstoffe, Surfactants, Co-Solventien, Stabilisatoren, Komplexbildnern, Geschmackskorrigentien, Antioxidantien, Salzen, Säuren, Basen oder Elektrolyten, wie beispielsweise Hydroxidionen, Cyanidionen und/oder Halogenidanionen wie Fluorid, Chlorid, Bromid oder Jodid.

Der Behälter (2) wird aus einem Mantel gebildet, der aus einer der oben beschriebenen Legierungen besteht. Der Behälter (2) weist vier verschiedene Zonen auf: den plan oder konkav nach innen gewölbten Boden (3), einen zylinderfömigen Bereich (4), der im oberen Drittel in den sich verjüngenden Hals (5) übergeht und schließlich in dem Wulst (6) endet, der die Öffnung (7) des Behälters umrandet.

Die Wandstärke des Behälters (2) beträgt in einer bevorzugten Ausführungsform zwischen 0,1 und 0,5 mm, bevorzugt zwischen 0,15 und 0,35 mm, ganz besonders bevorzugt ca. 0,19 bis 3,0 mm.

In einer bevorzugten Ausführungsform hält der Behälter (2) einem Berstdruck von mehr als 30000 hPa aus, bevorzugt mehr als 100000 hPa, ganz besonders bevorzugt mehr als 200000 hPa. Das Gewicht des Behälters (2) beträgt in einer bevorzugten Ausführungsform 5-15 g, in einer anderen 7 - 10 g und in wieder einer anderen 7,9 - 8,7 g. In einer gleichfalls bevorzugten Ausführungsform weist der Behälter (2) ein Volumen von 5 bis 50 ml auf. Andere Behälter weisen ein Volumen von 10 bis 20 ml und wieder andere von ca. 15 - 18 ml auf.

Im verschlossenen Zustand ist der Behälter (2) nach dem Befüllen mit der Arzneistoff-Formulierung und dem Treibmittel mit der Ventiltasse (8) dicht verschlossen.

In einer Ausführungsform besteht die Ventiltasse (8) ebenfalls aus korrosionsbeständigem Material. Bevorzugt handelt es sich dabei um die oben für den Behälter beschriebene Legierung und/oder Kunststoffmaterialien von geeigneter pharmazeutischer Qualität.

In einer anderen Ausführungsform besteht die Ventiltasse (8) aus Aluminium. In diesem Fall sind die Dichtung (10) und/oder das Ventil (9) derart ausgebildet, daß die Ventiltasse (8) selbst mit der im Inneren des Behälters befindlichen Flüssigkeit nicht in Berührung kommen kann.

Eine bevorzugte Ausführungsform der Ventiltasse (8) betrifft eine gemäß der GB 2324121, auf die hiermit in vollem Umfang Bezug genommen wird.

Im geschlossenen Zustand des Kanisters umcrimpt die Ventiltasse (8) den Behälter (2) an dessen Wulst (6). In bevorzugten Ausführungsformen dichtet eine Dichtung (10) die Ventiltasse (8) gegenüber dem Wulst (6) ab. Die Dichtung kann dabei ringförmig oder scheibenförmig sein. Bevorzugt ist sie scheibenförmig. Sie kann aus den aus dem Stand der Technik bekannten Materialien bestehen, die für die Verwendung von Arzneimittel-Formulierungen mit Fluorkohlenwasserstoffen als Treibmittel geeignet sind. Beispielsweise eigen sich hierfür Thermoplaste, Elastomere, Materialien wie Neopren, Isobutylen, Isopren, Butyl-Kautschuk, Buna-Kautschuk, Nitril-Kautschuk, Copolymere aus Ethylen und Propylen, Terpolymere aus Ethylen, Propylen und einem Dien, beispielsweise Butadien, oder fluorierte Polymere. Bevorzugtes Material sind Ethylen/Propylen-Dien-Terpolymere (EPDM).

Auf der zum Inneren des Behälters zugewandten Seite der Ventiltasse (8) ist ein Ventil (9) so ausgebildet, daß der Ventilstamm (12) durch die Ventiltasse (8) hindurch auf die andere Seite geht. Das Ventil (9) sitzt abdichtend in der zentralen Öffnung der Dichtung (10). Dichtung (10) und Ventil (9) zusammen dichten dabei die Ventiltasse (8) gegenüber dem Behälterinneren ab, so daß diese mit der Flüssigkeit in dem Behälter (2) nicht in Berührung kommen kann.

Das Ventil (9) ist derart aufgebaut, daß jedes Element, das mit der Flüssigkeit im Inneren des Behälters (2) in Berührung kommen kann, aus einem gegenüber dieser Flüssigkeit korrosionsbeständigem Material besteht. Zu diesen Elementen gehören beispielsweise die Feder oder Federn (11), der Ventilstamm (12), der durch die Öffnung (17) der Ventiltasse (8) von innen nach außen ragt, die Dosierkammer (13) und der Körper des Ventils (14). Die Feder (11) besteht aus Stahl, bevorzugt einem Edelstahl. Die weiteren Elemente des Ventils (9) können beispielsweise aus Stahl, der oben beschriebenen Legierung und/oder einem Kunststoff bestehen. Die Elemente (12), (13), und (14) bestehen bevorzugt aus einem Kunststoff, besonders bevorzugt aus einem Polyester, ganz besonders bevorzugt aus Polybutylenterephtalat.

Wie in Figur 1 dargestellt können eine oder mehrere weitere Dichtungen, z.B. die Dichtungen (15) und/oder (16), ausgebildet sein, die ein Entweichen von Flüssigkeit oder Gas aus dem Inneren des Behälters nach außen verhindern. Die Dichtung(en) kann (können) dabei auch so angeordnet sein, daß die Flüssigkeit im Inneren des Behälters außer mit der (den) Dichtung(en) selbst nur mit dem Behältermantel und dem Ventil in Kontakt kommt.

Die Dichtung (15) dichtet den optional vertikal beweglichen Ventilstamm an der Stelle ab, an der er die Ventiltasse (8) durchstößt. Die Dichtung (16) dichtet den Ventilstamm (12) im Inneren des Ventils gegenüber dem Ventilkörper (14) und/oder der Dosierkammer (13) ab. Damit verhindern die Dichtungen (15) und (16), daß eine Flüssigkeit oder ein Gas aus dem Inneren des Behälters entlang dem Außenmantel des Ventilstamms aus dem Kanister entweichen kann, bzw. über diesen Weg mit der Ventiltasse in Berührung kommt. Die Dichtungen (15) und (16) können aus dem gleichen Material wie die Dichtung (10) aufgebaut sein, bevorzugt einem Ethylen/Propylen-Dien-Terpolymer.

In einer Ausführungsform, bei der die Ventiltasse (8) nicht aus Aluminium, sondern aus einem der oben beschriebenen korrosionsbeständigen Materialien besteht, ist es nicht notwendig, daß die Dichtung (10) zusammen mit dem Ventil (9) die Ventiltasse vollständig gegenüber dem Behälterinneren isoliert. Daher ist es in diesem Fall auch nicht notwendig, daß die Dichtung (10) und das Ventil (9) einander abdichtend berühren. Gegebenenfalls besteht ein Spalt zwischen Dichtung (10) und Ventil (9). In einem solchen Fall sitzt die Dichtung (10) z. B. direkt auf der Unterseite der Ventiltasse (8) auf und dichtet den Rand der Ventiltasse (8) gegenüber dem Behälterwulst (6) ab. Die Dichtung (15) dichtet dann die Öffnung (17) in der Ventiltasse (8) gegenüber dem Inneren des Behälters ab.

Fig. 2 zeigt eine weitere Ausführungsform der Ventiltasse (8) mit eingebettetem Ventil (9). Diese Ausführungsform ist im großen und ganzen identisch mit der der Figur 1. Der wesentliche Unterschied besteht darin, daß die Dichtung (10) und die Dichtung (16) der Ausführungsform der Figur 2 zu der einen Dichtung (18) zusammengefaßt sind. Dichtung (18) ummantelt die Unterseite des Ventiltellers (8). Sie ist dergestalt, daß der Ventilkörper (14) in der Dichtung eingebettet vorliegt. Der Ventilstamm (12) passiert die Dichtung durch die Öffnung (19), die direkt unterhalb der Öffnung (17) der Ventiltasse (8) liegt. Die Öffnung (19) ist derart dimensioniert, daß sie den Ventilstamm (12) gegen die Ventiltasse (8) abdichtet. Das Dichtungsmaterial für die Dichtung (18) ist identisch mit dem für die Dichtung (10) beschriebenen.

Die Herstellung des erfindungsgemäße Behälters (2) geschieht in Analogie zu den nach aus dem Stand der Technik für Alukanister u.ä. bekannten Verfahren, nach denen der Behälter aus einem Blech des entsprechenden Materials, bzw. der entsprechenden Legierung herausgestanzt wird. Bei der vorliegenden Erfindung wird der Behälter (2) aus einem Blech der oben beschriebenen Legierungen aus Chrom (Cr), Nickel (Ni), Molybdän (Mo), Eisen (Fe) und Kohlenstoff (C) ausgestanzt oder aus einer Legierung, die zusätzlich Kupfer (Cu), Mangan (Mn) und Silicium (Si) enthält.

Der erfindungsgemäße Behälters (2) bzw. Kanister aus Behälters (2) und Ventiltasse (8) mit Ventil (9) ist besonders zur Verwendung mit Fluorkohlenwasserstoff-haltigen Treibgasformulierungen geeignet. Treibgasformulierungen, die bevorzugt zusammen mit der Erfindung verwendet werden können, sind in der WO 94/13262 offenbart, auf die hiermit in vollem Umfang Bezug genommen wird. Daraus besonders bevorzugt sind säurestabilisierte und/oder ethanolischen Treibgasformulierungen mit 1,1,2,2-Tetrafluorethan (TG 134a) und/oder 1,1,1,2,3,3,3-Heptafluorpropan (TG 227) als Treibgas, insbesondere solche die Ipatropiumbromid, Oxitropiumbromid, Albuterol, Tiotropiumbromid oder Fenoterol und als Wirkstoff enthalten.

Je nach Wirkstoff sind zum Stabilisieren anorganische und organische Säuren geeignet. Als Beispiele für anorganische Sauren werden neben Halogensäuren und weiteren Mineralsäuren genannt: Schwefelsäure, Salzsäure, Salpetersäure oder Phosphorsäure, als Beispiele organischer Säuren Ascorbinsäure oder Zitronensäure. Im Fall von Wirkstoffsalzen sind solche Säuren bevorzugt, deren Anion identisch ist mit dem des Wirkstoffsalzes. Für alle Wirkstoffe und Wirkstoffsalze ist generell Zitronensäure geeignet und auch am stärksten bevorzugt.

Der Gehalt an Säure ist derart, daß der pH-Wert der Formulierung zwischen 1,0 und 7,0, bevorzugt zwischen 2,0 und 5,0 und ganz besonders bevorzugt bei etwa 3,5 liegt. Im Fall von anorganischen Säuren befindet sich der bevorzugte Gehalt an Säure im Bereich von etwa 0,00002 bis 0,01 N. Im Fall von Ascorbinsäure befindet sich der bevorzugte Gehalt in etwa im Bereich von 0,0045 bis 5,0 mg/ml und im Fall der Zitronensäure im Bereich von 0,0039 bis 27,7 mg/ml.

Die Formulierungen können darüber hinaus Ethanol als Co-Solvens enthalten. Die bevorzugte Menge beträgt 1,0 bis 50,0 Gew.% der Formulierung,

Im folgenden sind beispielhaft bevorzugte Formulierungen, die in einer Kanister bzw. einem Behälter der oben beschriebenen Art gelagert werden können, aufgeführt:

### Beispiel 1

| | |
|---|---|
| Ipatropiumbromid Monohydrat | 0,001 - 2,5Gew.% |
| Ethanol, absolut | 0,001 - 50 Gew.% |
| TG 134a | 50,0 - 99,0 Gew.% |
| Anorganische Säure | 0,01 - 0,00002 Normal |
| Wasser | 0,0 - 5,0 Gew.%. |

### Beispiel 2

| | |
|---|---|
| Ipatropiumbromid Monohydrat | 0,001 - 2,5Gew.% |
| Ethanol, absolut | 0,001 - 50 Gew.% |
| TG 134a | 50,0 - 99,0 Gew.% |
| Ascorbinsäure | 0,00015 - 5,0 mg/ml |
| Wasser (gereinigt) | 0,0 - 5,0 Gew.%. |

### Beispiel 3

| | |
|---|---|
| Ipatropiumbromid Monohydrat | 0,0187 Gew.% |
| Ethanol, absolut | 15,0000 Gew.% |
| TG 134a | 84,4773 Gew.% |
| Zitronensäure | 0,0040 Gew.% |
| Wasser (gereinigt) | 0,5000 Gew.%. |
| Gesamt | 100,0000 Gew.% |

### Beispiel 4

| | |
|---|---|
| Ipatropiumbromid Monohydrat | 0,0374 Gew.% |
| Ethanol, absolut | 15,0000 Gew.% |
| TG 134a | 84,4586 Gew.% |
| Zitronensäure | 0,0040 Gew.% |
| Wasser (gereinigt) | 0,5000 Gew.%. |
| Gesamt | 100,0000 Gew.% |

### Beispiel 5

| | |
|---|---|
| Ipatropiumbromid Monohydrat | 0,0748 Gew.% |
| Ethanol, absolut | 15,0000 Gew.% |
| TG 134a | 84,4212 Gew.% |
| Zitronensäure | 0,0040 Gew.% |
| Wasser (gereinigt) | 0,5000 Gew.%. |
| Gesamt | 100,0000 Gew.% |

### Beispiel 6

| | |
|---|---|
| Fenoterol Hydrobromid | 0,192 Gew.% |
| Ethanol, absolut | 30,000 Gew.% |
| TG 134a | 67,806 Gew.% |
| Zitronensäure | 0,002 Gew.% |
| Wasser (gereinigt) | 2,000 Gew.%. |
| Gesamt | 100,0000 Gew.% |

Eine Methode, die Kanister mit der entsprechenden Formulierung zu füllen, können beispielsweise die "Dual Stage Pressure Fill-Methode", die "Single Stage Cold Fill-Methode" oder die "Single Stage Pressure Fill-Methode" sein.

## Patentansprüche

1. Kanister (1) für treibmittelbetriebene Dosierareosole, der aus einem Behälter (2) und einer Ventiltasse (8) mit eingebettetem Ventil (9) besteht und eine Treibgasformulierung mit einem Fluorkohlenwasserstoff als Treibmittel enthält, **dadurch gekennzeichnet, daß** die Treibmittelformulierung eine Säure zum Einstellen eines pH-Werts von 1,0 bis 7,0 und einen Wirkstoff ausgewählt aus der Gruppe Ipratropium Bromide, Oxitropium Bromide, Albuterol, Tiotropium Bromide oder Fenoterol enthält und dass der Behälter aus einer Legierung mit einem Anteil an Eisen von 40,0 - 53,0%, Nickel 23,0 - 28,0%, Chrom 19,0 - 23,0%, Molybdän 4,0 - 5,0%, Mangan 0,0 - 2,0%, Cupfer 1,0 - 2,0%, Silicium 0,0 - 1,0%, Phosphor 0,0 - 0,045%, Schwefel 0,0 - 0,035% und Kohlenstoff 0,0 - 0,020% besteht.

2. Kanister nach Anspruch 1, **dadurch gekennzeichnet, daß** der Behälter aus einer Legierung mit folgender Zusammensetzung besteht Chrom 19,0 - 21,0%, Nickel 24,0 - 26,0%, Molybdän 4,0 - 5,0%, Kupfer 1,0 - 2,0%, Mangan bis zu 2,0%, Silicium bis zu 0,5% und Kohlenstoff bis zu 0,02% und mit Eisen als im wesentlichen restlichem Bestandteil.

3. Kanister (1) für treibmittelbetriebene Dosierareosole, der aus einem Behälter (2) und einer Ventiltasse (8) mit eingebettetem Ventil (9) besteht und eine Treibgasformulierung mit einem Fluorkohlenwasserstoff als Treibmittel enthält, **dadurch gekennzeichnet, daß** die Treibmittelformulierung eine Säure zum Einstellen eines pH-Werts von 1,0 bis 7,0 und einen Wirkstoff ausgewählt aus der Gruppe Ipratropium Bromide, Oxitropium Bromide, Albuterol, Tiotropium Bromide oder Fenoterol enthält und dass der Behälter aus Eisen 60,0 - 72,0%, Nickel 9,0 - 13,0%, Chrom 17,0 - 21,0%, Molybdän 2,0 - 3,0%, Mangan 0,0 -1,5%, Silicium 0,0 - 1,5%, Phosphor 0,0 - 0,04%, Schwefel 0,0 - 0,04% und Kohlenstoff 0 - 0,03% besteht.

4. Kanister (1) für treibmittelbetriebene Dosierareosole, der aus einem Behälter (2) und einer Ventiltasse (8) mit eingebettetem Ventil (9) besteht und eine Treibgasformulierung mit einem Fluorkohlenwasserstoff als Treibmittel enthält, **dadurch gekennzeichnet, daß** die Treibmittelformulierung eine Säure zum Einstellen eines pH-Werts von 1,0 bis 7,0 und einen Wirkstoff ausgewählt aus der Gruppe Ipratropium Bromide, Oxitropium Bromide, Albuterol, Tiotropium Bromide oder Fenoterol enthält und dass der Behälter aus einer Legierung aus 16,5 - 18,5 % Chrom, 11,0 - 14,0% Nickel, 2,0 - 2,5% Molybdän, maximal 0,03% Kohlenstoff und Eisen als restlichem Bestandteil besteht.

5. Kanister nach einem der Ansprüche 1 bis 4, **dadurch gekennzeichnet, daß** die Treibmittelformulierung eine Säure zum Einstellen eines pH-Werts von 2,0 bis 5,0 enthält.

6. Kanister nach Anspruch 5, **dadurch gekennzeichnet, daß** die Säure Schwefelsäure, Salzsäure, Salpetersäure, Phosphorsäure, Ascorbinsäure oder Zitronensäure, bevorzugt Zitronensäure ist.

7. Kanister nach einem der Ansprüche 1 bis 6, **dadurch gekennzeichnet, daß** das Treibmittel TG 134a und/oder TG 227 ist.

8. Kanister nach einem der Ansprüche 1 bis 7, **dadurch gekennzeichnet, daß** die Formulierung Ethanol als Co-Solvens enthält.

9. Kanister (1) für treibmittelbetriebene Dosierareosole bestehend aus einem Behälter (2) und einer Ventiltasse (8) mit eingebettetem Ventil (9) **dadurch gekennzeichnet, daß** der Behälter aus einer Legierung mit einem Anteil an Eisen von 40,0 - 53,0%, Nickel 23,0 - 28,0%, Chrom 19,0 - 23,0%, Molybdän 4,0 - 5,0%, Mangan 0,0 - 2,0%, Cupfer 1,0 - 2,0%, Silicium 0,0 - 1,0%, Phosphor 0,0 - 0,045%, Schwefel 0,0 - 0,035% und Kohlenstoff 0,0 - 0,020% besteht.

10. Kanister nach Anspruch 9, **dadurch gekennzeichnet, daß** der Behälter aus einer Legierung mit folgender Zusammensetzung besteht Chrom 19,0 - 21,0%, Nickel 24,0 - 26,0%, Molybdän 4,0 - 5,0%, Kupfer 1,0 - 2,0%, Mangan bis zu 2,0%, Silicium bis zu 0,5% und Kohlenstoff bis zu 0,02%, und mit Eisen als im wesentlichen restlichem Bestandteil.

11. Kanister (1) für treibmittelbetriebene Dosierareosole bestehend aus einem Behälter (2) und einer Ventiltasse (8) mit eingebettetem Ventil (9) **dadurch gekennzeichnet, daß** der Behälter aus einer Legierung besteht, die folgende Zusammensetzung aufweist: Eisen 60,0 - 72,0%, Nickel 9,0 - 13,0%, Chrom 17,0 - 21,0%, Molybdän 2,0 - 3,0%, Mangan 0,0 -1,5%, Silicium 0,0 - 1,5%, Phosphor 0,0 - 0,04%, Schwefel 0,0 - 0,04% und Kohlenstoff 0 - 0,03%.

12. Kanister (1) für treibmittelbetriebene Dosierareosole bestehend aus einem Behälter (2) und einer Ventiltasse (8) mit eingebettetem Ventil (9) **dadurch gekennzeichnet, daß** der Behälter aus einer Legierung aus 16,5 - 18,5 % Chrom, 11,0 - 14,0% Nickel, 2,0 - 2,5% Molybdän, maximal 0,03% Kohlenstoff und Eisen als restlichem Bestandteil besteht.

13. Kanister nach einem der Ansprüche 1 bis 12, **dadurch gekennzeichnet, daß** die Ventiltasse (8) aus Aluminium besteht und mit einer Dichtung (10) und/oder (18) gegenüber dem Behälterinnenraum verschlossen ist.

14. Kanister nach einem der Ansprüche 1 bis 13, **dadurch gekennzeichnet, daß** das Ventil (9) eine oder mehrere Edelstahlfeder(n) (11), einen Ventilstamm (12), die Dosierkammer (13) und einen Ventilkörper (14) beinhaltet, wobei der Ventilstamm (12), die Dosierkammer (13) und/oder der Ventilkörper (14) aus Stahl, der Legierung nach einem der Ansprüche 1 bis 4 oder 9 bis 12 und/oder einem Kunststoff bestehen.

15. Kanister nach einem der Ansprüche 1 bis 14, **dadurch gekennzeichnet, daß** die Feder(n) (11) aus einem Edelstahl und der Ventilstamm (12), die Dosierkammer (13) und der Körper des Ventils (14) aus Polybutylenterephtalat bestehen.

16. Kanister nach einem der Ansprüche 1 bis 15, **dadurch gekennzeichnet, daß** der Ventilstamm (12) durch eine Dichtung (15) oder (18) gegenüber der Ventiltasse (8) abgedichtet ist.

17. Kanister nach einem der Ansprüche 1 bis 16, **dadurch gekennzeichnet, daß** die Dichtung (10) und/oder Dichtung (15) und/oder Dichtung (18) aus einem Ethylen/Propylen-Dien-Terpolymer besteht (bestehen).

18. Kanister nach einem der Ansprüche 1 bis 17, **dadurch gekennzeichnet, daß** die Ventiltasse (8) aus der gleichen Legierung wie der Behälter (2) besteht.

19. Kanister nach einem der Ansprüche 1 bis 18, **dadurch gekennzeichnet, daß** der Behälter einem Berstdruck von mehr als 30000 hPa, bevorzugt mehr als 100000 hPa standhält.

20. Kanister nach einem der Ansprüche 1 bis 19, **dadurch gekennzeichnet, daß** der Behälter einen Berstdruck von mehr als 200000 hPa standhält.

21. Kanister nach einem der Ansprüche 1 bis 20, **dadurch gekennzeichnet, daß** der Behälter eine Wandstärke von 0,1 bis 0,5 mm, bevorzugt 0,15 bis 0,35 mm, aufweist.

22. Kanister nach einem der Ansprüche 1 bis 21, **dadurch gekennzeichnet, daß** der Behälter eine Wandstärke von 0,19 bis 3,0 mm aufweist.

23. Behälter für einen Kanister (1) für treibmittelbetriebene Dosieraerosole, der eine Treibgasformulierung mit einem Fluorkohlenwasserstoff als Treibmittel enthält, **dadurch gekennzeichnet, daß** die Treibmittelformulierung eine Säure zum Einstellen eines pH-Werts von 1,0 bis 7,0 enthält und einen Wirkstoff ausgewählt aus der Gruppe Ipratropium Bromide, Oxitropium Bromide, Albuterol, Tiotropium Bromide oder Fenoterol und dass der Behälter aus einer Legierung nach einem der Ansprüche 1 bis 4 besteht.

24. Behälter nach Anspruch 23, **dadurch gekennzeichnet, daß** die Treibmittelformulierung eine Säure zum Einstellen eines pH-Werts von 2,0 bis 5,0 enthält.

25. Behälter nach einem der Ansprüche 32 oder 24, **dadurch gekennzeichnet, daß** die Säure Schwefelsäure, Salzsäure, Salpetersäure, Phosphorsäure, Ascorbinsäure oder Zitronensäure, bevorzugt Zitronensäure ist.

26. Behälter nach einem der Ansprüche 23 bis 25, **dadurch gekennzeichnet, daß** das Treibmittel 1TG 134a und/oder TG 227 ist.

27. Behälter nach einem der Ansprüche 23 bis 26, **dadurch gekennzeichnet, daß** die Formulierung Ethanol als Co-Solvens enthält.

28. Behälter für einen Kanister (1) für treibmittelbetriebene Dosieraerosole, **dadurch gekennzeichnet daß** der Behälter aus einer Legierung nach einem der Ansprüche 9 bis 12 besteht.

29. Behälter nach einem der Ansprüche 23 bis 28, **dadurch gekennzeichnet, daß** der Behälter einen Berstdruck von mehr als 30000 hPa, bevorzugt mehr als 100000 hPa standhält.

30. Behälter nach einem der Ansprüche 23 bis 29, **dadurch gekennzeichnet, daß** der Behälter einen Berstdruck von mehr als 200000 hPa standhält.

31. Behälter nach einem der Ansprüche 23 bis 30, **dadurch gekennzeichnet, daß** der Behälter eine Wandstärke von 0,1 bis 0,5 mm, bevorzugt 0,15 bis 0,35 mm, aufweist.

32. Behälter nach einem der Ansprüche 23 bis 31, **dadurch gekennzeichnet, daß** der Behälter eine Wandstärke von 0,19 bis 3,0 mm aufweist.

33. Verfahren zur Herstellung eines Behälters (2) nach einem der Ansprüche 23 bis 32 oder eines Kanisters (1) nach einem der Ansprüche 1 bis 22, **dadurch gekennzeichnet, daß** der Behälter (2) aus einem Blech herausgestanzt wird, das aus einer Legierung nach einem der Ansprüche 1 bis 4 oder 9 bis 12 besteht.

34. Verwendung eines Kanisters nach den Ansprüchen 9 bis 12 oder eines Behälters nach Anspruch 28 in einem Inhalator und/oder zur Lagerung von Wirkstoffformulierungen, die 1,1,2,2-Tetrafluorethan und/oder 1,1,1,2,3,3,3-Heptafluorpropan als Treibmittel enthalten.

35. Verwendung nach Anspruch 34, **dadurch gekennzeichnet, daß** die Formulierung Säure zum Stabilisieren, Ethanol als Co-Solvens und Ipatropium Bromid, Oxitropium Bromid, Tiotropium Bromid, Albuterol oder Fenoterol als Wirkstoff enthält.

36. Verwendung nach einem der Ansprüche 34 oder 35, **dadurch gekennzeichnet, daß** die Formulierung Säure zum Stabilisieren, Ethanol und Ipatropium Bromid, Oxitropium Bromid oder Tiotropium Bromid als Wirkstoff enthält.

37. Verwendung nach einem der Ansprüche 34 bis 36, **dadurch gekennzeichnet, daß** die Formulierung Zitronensäure enthält.

38. Verwendung nach einem der Ansprüche 34 oder 36, **dadurch gekennzeichnet, daß** die Formulierung eine Mineralsäure enthält.

39. Verwendung nach einem der Ansprüche 34 oder 36, **dadurch gekennzeichnet, daß** die Formulierung Salzsäure enthält.
